(19) **Europäisches Patentamt / European Patent Office / Office européen des brevets**

(11) **EP 2 794 104 B1**

(12) **FASCICULE DE BREVET EUROPEEN**

(45) Date de publication et mention
de la délivrance du brevet:
**17.06.2020 Bulletin 2020/25**

(21) Numéro de dépôt: **12813403.8**

(22) Date de dépôt: **27.11.2012**

(51) Int Cl.:
*B01J 37/02* [(2006.01)]    *B01J 37/28* [(2006.01)]
*B01J 23/882* [(2006.01)]    *B01J 23/75* [(2006.01)]
*B01J 27/19* [(2006.01)]    *B01J 27/14* [(2006.01)]
*B01J 23/28* [(2006.01)]    *C07C 5/10* [(2006.01)]
*C07C 13/18* [(2006.01)]    *C07C 5/00* [(2006.01)]
*C10G 45/08* [(2006.01)]    *B01J 37/20* [(2006.01)]
*B01J 29/90* [(2006.01)]    *B01J 38/52* [(2006.01)]
*B01J 38/62* [(2006.01)]

(86) Numéro de dépôt international:
**PCT/FR2012/000487**

(87) Numéro de publication internationale:
**WO 2013/093228 (27.06.2013 Gazette 2013/26)**

(54) **PROCEDE DE PREPARATION D'UN CATALYSEUR UTILISABLE EN HYDROTRAITEMENT ET HYDROCONVERSION**

PROZESS ZUR HERSTELLUNG EINES KATALYSATORS ZUR HYDROCONVERSION

PROCESS OF PREPARATION OF A HYDROTREATMENT/HYDROCONVERSION CATALYST

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2011 FR 1104026**

(43) Date de publication de la demande:
**29.10.2014 Bulletin 2014/44**

(73) Titulaires:
• **IFP Energies nouvelles**
 **92500 Rueil-Malmaison (FR)**
• **Total Raffinage France**
 **92400 Courbevoie (FR)**

(72) Inventeurs:
• **GUICHARD, Bertrand**
 **F-38140 Izeaux (FR)**
• **SIMON, Laurent**
 **F-69100 Villeurbanne (FR)**

• **LOPEZ, Sylvie**
 **F-69007 Lyon (FR)**
• **DE GRANDI, Valentina**
 **B-1030 Schaerbeek (BE)**
• **MINOUX, Delphine**
 **B-1400 Nivelles (BE)**
• **DATH, Jean-Pierre**
 **B-7970 Beloeil (BE)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**EP-A1- 1 900 430    WO-A1-2005/035691**
**WO-A1-2006/077326    WO-A1-2009/126319**
**WO-A1-2011/080407**

## Description

L'invention concerne un procédé de préparation d'un catalyseur comprenant un support et une fonction hydro-déshydrogénante, ce procédé comprenant l'introduction d'un additif qui est un succinate de dialkyle C1-C4.

ART ANTERIEUR

L'utilisation d'additif pour améliorer l'activité d'un catalyseur séché, calciné ou régénéré est bien connue de l'homme du métier. Le brevet EP0870003 enseigne ainsi que l'utilisation de composés ayant au moins 2 fonctions hydroxyle ou des polyéthers correspondants, introduits en présence de solvant (alcools ou eau) par imprégnation d'un précurseur catalytique calciné ou régénéré conduit à un gain d'activité à condition de préserver au moins 50% de l'additif et préférentiellement 70% sur le catalyseur après le traitement thermique final. Ce protocole de préparation ne concerne pas l'utilisation d'acide carboxylique.

Le brevet WO2006/077326 propose l'utilisation d'un catalyseur comprenant des métaux des groupes VIB et VIII, un oxyde réfractaire comme support, et un composé organique comportant au moins 2 fonctions ester carboxylique de formule R1-O-CO-R2-CO-O-R1 ou R1-CO-O-R2-O-CO-R1 dans laquelle chaque R1 représente indépendamment un groupe alkyle en C1 à C18, alcényle C2 à C18, aryle C6 à C18, cycloalkyle en C3 à C8, alkylaryle ou arylalkyle en C7 à C20, ou les 2 groupes R1 forment conjointement un groupe divalent en C2 à C18, et R2 représente un groupe alkylène en C1 à C18, arylène en C6 à C18, cycloalkylène en C3 à C7, ou une combinaison de ceux-ci, la chaîne carbonée des groupes hydrocarbonés représentés par R1 et R2 pouvant contenir ou porter un ou plusieurs hétéroatomes choisis parmi N,S et O, et chacun des groupes R1 et R2 pouvant porter un ou plusieurs substituants de formule -C(=O)O-R1 ou -O-C(=O)-R1 où R1 a la signification indiquée ci-dessus. Un mode préféré utilise le succinate de dialkyle C1-C4, et en particulier le succinate de diméthyle qui est exemplifié. Ces composés peuvent être introduits en présence d'un solvant (une liste importante de solvants est citée parmi laquelle les acides carboxyliques).

La demande de brevet WO11/080407 décrit l'incorporation simultanée d'un succinate de dialkyle C1-C4 en présence d'acide acétique. La combinaison des deux composés permet d'augmenter considérablement les performances catalytiques sur des catalyseurs séchés.

Le brevet WO05/035691. revendique un procédé d'activation permettant de diminuer la teneur en phase cristallisée de type $CoMoO_4$ présente sur les catalyseurs régénérés comprenant des oxydes des métaux des groupes VIII et VIB. Ce procédé décrit la mise en contact d'un catalyseur régénéré avec un acide et un additif organique. L'imprégnation simultanée de la combinaison acide citrique (CA) et polyéthylène glycol (PEG) a été exemplifiée sur un catalyseur régénéré..Les exemples montrent l'intérêt d'un couple acide citrique / PEG.

Les acides carboxyliques sont décrits dans le brevet EP0482817 pour la préparation de catalyseurs séchés ayant une activité élevée, c'est-à-dire non calcinés et sans autre ajout de molécules organiques.

DESCRIPTION DE L'INVENTION

La présente invention concerne un procédé de préparation d'un catalyseur et son utilisation pour l'hydrotraitement et l'hydroconversion. Il est obtenu une amélioration des performances catalytiques (notamment de l'activité catalytique) par rapport aux catalyseurs de l'art antérieur. En effet, il a été mis en évidence que l'utilisation d'une méthode particulière de préparation, utilisant au moins un succinate de dialkyle C1-C4, et en particulier de diméthyle succinate, et au moins un acide carboxylique autre que l'acide acétique, en présence éventuellement d'acide acétique, sur un précurseur catalytique séché, calciné ou régénéré conduit de façon surprenante à une activité catalytique nettement améliorée.

Le catalyseur présente un spectre Raman avec les bandes à 990 et/ou 974 $cm^{-1}$ caractéristiques d'au moins un hétéropolyanion de Keggin et éventuellement des bandes caractéristiques dudit succinate. Les raies correspondant au succinate sont présentes ou non suivant les conditions du procédé, notamment les conditions des séchages.

L'invention concerne également le catalyseur activé et son utilisation dans un procédé d'hydrotraitement et/ou d'hydroconversion. Le catalyseur activé est obtenu par sulfuration du catalyseur décrit dans la présente demande.

Le catalyseur comprend un support et une fonction hydro-déshydrogénante et du phosphore. La fonction hydro-déshydrogénante comprend au moins un élément du groupe VIB (de préférence molybdène et/ou tungstène) et éventuellement (et de préférence) au moins un élément du groupe VIII (de préférence cobalt et/ nickel) . De préférence, la fonction hydro-déshydrogénante comprend du molybdène et du cobalt et/ou du nickel.

Le catalyseur obtenu possède un spectre Raman caractéristique regroupant :

1) des bandes caractéristiques du ou des hétéropolyanions de type Keggin $PXY_{11}O_{40}^{x-}$ et/ou $PY_{12}O_{40}^{x-}$ où Y est un métal du groupe VIB et X un métal du groupe VIII.

D'après Griboval, Blanchard, Payen, Fournier, Dubois dans Catalysis Today 45 (1998) 277 fig. 3 e), les bandes principales de la structure $PCoMo_{11}O_{40}{}^{x-}$ sont sur catalyseur séché à 232, 366, 943, 974 cm$^{-1}$ et d'après M. T. Pope "Heteropoly and Isopoly oxometalates", Springer Verlag, p 8, ces bandes ne sont pas caractéristiques de la nature de l'atome X ou Y, mais bien de la structure de l'hétéropolyanion. La bande la plus intense caractéristique de ce type d'hétéropolyanion de Keggin lacunaire se situe à 974 cm$^{-1}$.

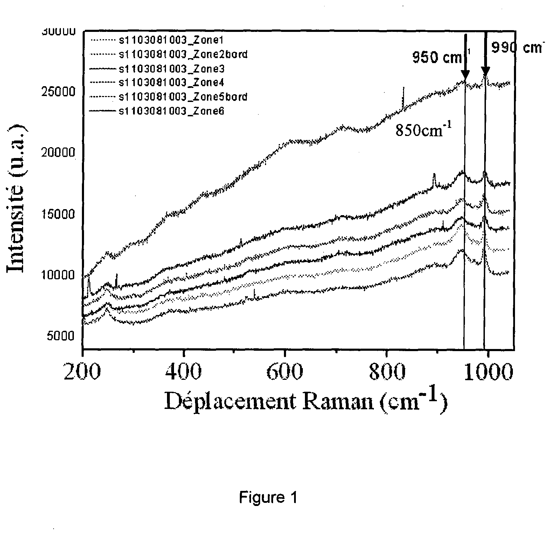

D'après Griboval, Blanchard, Gengembre, Payen, Fournier, Dubois, Bernard, Journal of Catalysis 188 (1999) 102, fig. 1 a), les bandes principales de $PMo_{12}O_{40}{}^{x-}$ sont à l'état massique de l'hétéropolyanion, par exemple avec du cobalt en contre ion à 251, 603, 902, 970, 990 cm$^{-1}$. La bande la plus intense caractéristique de cet hétéropolyanion de Keggin se situe à 990 cm$^{-1}$. M. T. Pope "Heteropoly and Isopoly oxometalates", Springer Verlag, p 8, nous enseigne également que ces bandes ne sont pas caractéristiques de la nature de l'atome X ou Y, mais bien de la structure de l'hétéropolyanion de Keggin, complet, lacunaire ou substitué.

2) éventuellement des bandes caractéristiques du (des) succinate(s) de dialkyle utilisé(s). Le spectre Raman du succinate de diméthyle constitue une empreinte univoque de cette molécule. Dans la zone spectrale 300-1800 cm$^{-1}$, ce spectre se caractérise par la série de bandes suivantes (seules les bandes les plus intenses sont reportées, en cm$^{-1}$) : 391, 853 (bande la plus intense), 924, 964, 1739 cm$^{-1}$. La bande la plus intense caractéristique du succinate de diméthyle est à 853 cm$^{-1}$. Le spectre du succinate de diéthyle comporte dans la zone spectrale considérée les bandes principales suivantes : 861 (bande la plus intense), 1101, 1117 cm$^{-1}$. De même pour le succinate de dibutyle : 843, 1123, 1303, 1439,1463 cm$^{-1}$ et pour le succinate de diisopropyle : 833, 876, 1149, 1185, 1469 (bande la plus intense), 1733 cm$^{-1}$

Les spectres Raman ont été obtenus avec un spectromètre de type Raman dispersif équipé d'un laser argon ionisé (514 nm). Le faisceau laser est focalisé sur l'échantillon à l'aide d'un microscope équipé d'un objectif x50 longue distance de travail. La puissance du laser au niveau de l'échantillon est de l'ordre de 1 mW. Le signal Raman émis par l'échantillon est collecté par le même objectif et est dispersé à l'aide d'un réseau 1800 tr/mn puis collecté par un détecteur CCD. La résolution spectrale obtenue est de l'ordre de 0,5 cm$^{-1}$. La zone spectrale enregistrée est comprise entre 300 et 1800 cm$^{-1}$. La durée d'acquisition a été fixée à 120 s pour chaque spectre Raman enregistré.

Le succinate de dialkyle est avantageusement le succinate de diméthyle, le succinate de dibutyle, le succinate de diisopropyle.

De préférence, le succinate de dialkyle utilisé est le succinate de diméthyle, et le catalyseur possède dans son spectre les bandes Raman principales à 990 et/ou 974 cm$^{-1}$ caractéristique(s) du (des) hétéropolyanion(s) de Keggin, et 853 cm$^{-1}$ caractéristique du succinate de diméthyle.

## Procédé de préparation selon l'invention

Plus précisément, l'objet de l'invention est un procédé de préparation d'un catalyseur à partir d'un précurseur catalytique comprenant un support à base d'alumine et/ou de silice-alumine et/ou de zéolite, et comprenant au moins un élément du groupe VIB et éventuellement au moins un élément du groupe VIII, ledit procédé comprenant l'imprégnation dudit précurseur par une solution d'un succinate de dialkyle C1-C4, caractérisé en ce qu'il comporte les étapes suivantes :

1) imprégnation (étape 1) dudit précurseur séché , calciné ou régénéré, avec au moins une solution contenant au moins un acide carboxylique autre que l'acide acétique, puis maturation et séchage à une température inférieure à 200°C, éventuellement suivi d'un traitement thermique à une température inférieure à 350°C, de préférence inférieure à 300°C,

2) suivie d'une imprégnation (étape 2) avec une solution contenant au moins un succinate de dialkyle C1-C4 puis maturation et séchage à une température inférieure à 200°C, sans étape de calcination ultérieure et, le précurseur catalytique et/ou la solution de l'étape 1 et/ou la solution de l'étape 2 contient du phosphore.

De préférence, le précurseur catalytique contient du phosphore.

De préférence, la première étape (étape 1) est une imprégnation du précurseur catalytique séché, calciné ou régénéré avec une solution comprenant un acide carboxylique, éventuellement de l'acide acétique et éventuellement du phosphore, de préférence dilués dans un solvant. Cette imprégnation est suivie d'une étape de maturation dudit précurseur catalytique imprégné, puis d'un séchage à une température inférieure à 200°C.

De préférence, la seconde étape (étape 2) est une imprégnation du précurseur catalytique issu de l'étape 1 par une solution contenant du succinate de dialkyle C1-C4 en présence ou non d'un composé contenant du phosphore et avec éventuellement (et de préférence) de l'acide acétique, suivie d'une étape de maturation dudit précurseur catalytique issu de l'étape 1 imprégné, puis d'un séchage à une température inférieure à 200°C sans étape de calcination (traitement thermique sous air) ultérieure.

Le catalyseur obtenu est de préférence soumis à une étape de sulfuration pour être activé avant son utilisation.

Le précurseur catalytique de l'étape 1 (sa composition et les traitements thermiques seront décrits en détail plus loin dans le texte).

Le précurseur catalytique comprend un support à base d'alumine, et/ou de silice-alumine, et/ou de zéolithe, et au moins un élément du groupe VIB, éventuellement au moins un élément du groupe VIII et éventuellement du phosphore. Ledit (lesdits) élément(s) a été introduit de préférence par imprégnation dudit support avec au moins une solution dudit(desdits) élément(s). L'imprégnation est suivie d'un séchage à une température inférieure à 200°C, et le plus souvent à 180°C. Il est obtenu un "précurseur catalytique séché".

Le séchage peut être suivi d'une suivi d'une calcination sous atmosphère oxydante à une température d'au moins 350°C, on appellera le produit "précurseur catalytique calciné". La température de la calcination est inférieure à 600°C et le plus souvent inférieure à 550°C.

Le précurseur catalytique peut être un catalyseur régénéré . Il a été utilisé par exemple dans des procédés d'hydrotraitement et/ou d'hydroconversion. Le catalyseur , après utilisation, est dit "usé" et sa désactivation est telle qu'une régénération est nécessaire. On appellera "précurseur catalytique régénéré" un catalyseur qui a été régénéré.

La régénération permet la combustion du carbone déposé sur le catalyseur lors de son utilisation industrielle. Elle peut être réalisée par tous les moyens connus de l'homme du métier. La régénération est en général réalisée à des températures comprises entre 350 et 550°C, et le plus souvent entre 400 et 520°C, ou entre 420 et 520°C, ou encore entre 450 et520°C, des températures inférieures à 500°C étant souvent avantageuses.

Le procédé de préparation d'un catalyseur selon l'invention comporte les étapes de préparations suivantes :

1) au moins une étape d'imprégnation par une solution comprenant au moins un acide carboxylique qui de manière préférée est l'acide citrique, éventuellement au moins un composé de phosphore, et éventuellement l'acide acétique, suivie d'une étape de maturation et d'un séchage à une température inférieure à 200°C, suivi éventuellement d'un traitement thermique à une température inférieure à 350°C. De préférence, le séchage est effectué à 100-180°C.

2) puis au moins une étape d'imprégnation par une solution d'imprégnation comprenant au moins un succinate de dialkyle C1-C4, éventuellement au moins un composé de phosphore, notamment si le phosphore n'a pas été introduit par imprégnation en totalité auparavant, et éventuellement (et de préférence) l'acide acétique, suivie d'une étape de maturation, et d'une étape de séchage à une température inférieure à 200°C, sans étape de calcination ultérieure. De préférence, le séchage est effectué à 50-160°C.

De préférence, le produit obtenu à l'issue de l'étape 2) subit une étape de sulfuration. L'invention concerne également le catalyseur sulfuré.

D'autres modes de réalisation peuvent être envisagés, qui restent dans l'invention, par exemple après le séchage de l'étape 1, le précurseur catalytique subit un traitement thermique au-dessus de la température de séchage initiale et en-dessous de la température de calcination (qui est souvent d'au moins 350°C), de préférence, la température du traitement est inférieure à 300°C. Ce mode de réalisation permet notamment d'envisager l'emploi d'acide carboxylique lourd (point d'ébullition élevé).

Ces procédés de préparation simples et rapides, avec des étapes unitaires ne dépassant pas quelques heures, permettent ainsi une meilleure productivité à l'échelle industrielle que les procédés présentés dans l'art antérieur.

Ainsi que cela sera décrit ultérieurement, le procédé selon l'invention est réalisé de préférence avec les modes suivants pris seuls ou en combinaison :

- le précurseur catalytique contient la totalité de la fonction hydrogénante (c'est-à-dire la totalité des éléments du groupe GVIB et s'ils sont présents, la totalité des éléments du groupe VIII), qui de préférence a été introduite par imprégnation lors de la préparation du précurseur catalytique frais

- lors de la préparation du catalyseur frais, la totalité du phosphore est introduite dans le précurseur catalytique, les solutions d'imprégnation des étapes 1 et 2 ne contiennent pas de phosphore

- le succinate de dialkyle est le succinate de diméthyle

- l'acide carboxylique est l'acide citrique

- les étapes 1) et/ou 2) sont réalisées en présence d'eau et/ou d'éthanol, et en particulier l'étape 1)

- l'étape 2) est réalisée en présence d'acide acétique

- les étapes de maturation sont réalisées à une température entre 17 et 60°C

- le séchage du produit imprégné dans l'étape 1 est réalisé à une température comprise entre 100 et 180°C

- le séchage du produit imprégné dans l'étape 2 est réalisé à une température comprise entre 50 et 160°C.

**Le précurseur catalytique**

Le précurseur catalytique séché ou calciné du procédé de préparation selon l'invention ou ayant conduit au précurseur catalytique régénéré peut être préparé par toutes les méthodes bien connues de l'homme du métier.

Le support

Le support dudit précurseur catalytique est à base d'alumine et/ou de silice-alumine et/ou de zéolithe.

De façon préférée, il contient uniquement de l'alumine et/ou de la silice-alumine et/ou de la zéolithe avec éventuellement le(s) métal (métaux) et/ou le(s) dopant(s) qui ont été introduits en-dehors des imprégnations (introduits par ex lors de la préparation - malaxage, peptisation... du support ou de sa mise en forme). Le support peut contenir un ou des dopants tels que du bore, du phosphore ou du fluor. Le support peut également contenir tout autre élément connu de l'homme du métier qui peut être introduit dans le support en-dehors des imprégnations.

Dans un mode de réalisation, le support contient généralement plus de 10%, voire 25 %, voire plus de 35 % et de préférence plus de 50 % poids d'alumine. De préférence, le support est constitué d'alumine. De préférence, l'alumine est l'alumine gamma et de préférence ledit support est constitué d'alumine gamma.

Dans un autre mode de réalisation, le support est une silice-alumine contenant de préférence au plus de 1%, voire 10%, voire au plus de 25 %, de préférence au plus de 35 % et de manière encore plus préférée au moins (ou plus de) 50% poids d'alumine. La teneur en silice dans le support est d'au plus 99% poids, voire inférieure à 90%, de préférence inférieure ou égale à 65 % poids, et de manière plus préférée inférieure ou égale à 50 % poids. De préférence, le support est constitué de silice-alumine.

Dans un autre mode de réalisation, le support du précurseur catalytique contient une ou plusieurs zéolithe(s) en sus de l'alumine et/ou de la silice-alumine, dans une proportion généralement inférieure à 50%poids, de manière préférée inférieure à 45%poids et de manière très préférée inférieure à 40%poids.

La zéolithe ou le mélange de zéolithes contenu dans le support du précurseur catalytique utilisé selon l'invention, comprend au moins une série de canaux dont l'ouverture est définie par un anneau contenant 12 atomes d'oxygène (12MR) Ladite zéolithe est choisie parmi les zéolithes définies dans la classification "Atlas of Zeolite Structure Types", Ch. Baerlocher, L. B. Mc Cusker, D.H. Oison, 6ème Edition, Elsevier, 2007, Elsevier" présentant au moins une série de canaux dont l'ouverture de pores est définie par un anneau contenant 12 atomes d'oxygène. La zéolithe initialement utilisée, avant d'être modifiée, contient avantageusement, en plus d'au moins une série de canaux dont l'ouverture de pores est définie par un anneau contenant 12 atomes d'oxygène (12MR), au moins une série de canaux dont l'ouverture de pores est définie par un anneau contenant 8 atomes d'oxygène (8 MR) et / ou au moins une série de canaux dont l'ouverture de pores est définie par un anneau contenant 10 atomes d'oxygène (10 MR). De manière préférée, les zéolithes de type structural FAU et BEA modifiées ou non sont utilisées. Elles sont mélangées à l'alumine et/ou la silice-alumine lors de la mise en forme du support. De manière préférée, la ou les zéolithes utilisées ont été modifiées pour permettre la création de mésoporosité par désalumination et/ou par désilication ou par toutes autres méthodes connues par l'homme du métier.

Le support constitué d'alumine et/ou de silice-alumine et/ou de zéolithe peut être mis en forme par toute technique connue de l'homme du métier. La mise en forme peut être réalisée par exemple par extrusion, par pastillage, par la méthode de la coagulation en goutte (oil-drop), par granulation au plateau tournant ou par toute autre méthode bien connue de l'homme du métier.

La fonction hydrogénante

Le précurseur catalytique contient une fonction hydro-déshydrogénante. Elle est assurée par au moins un élément du groupe VIB et éventuellement par au moins un élément du groupe VIII, et de préférence au moins un élément du groupe VIII et au moins un élément du groupe VIB.

La teneur totale en éléments hydro-déshydrogénants est avantageusement supérieure à 6% poids d'oxyde par rapport au poids total du catalyseur. Les éléments du groupe VIB préférés sont le molybdène et le tungstène, et en particulier le molybdène. Les éléments du groupe VIII préférés sont des éléments non nobles et en particulier le cobalt et le nickel.

Avantageusement, la fonction hydro-déshydrogénante comprend (et de préférence est constituée de) du molybdène, du nickel et/ou du cobalt.

Avantageusement, la fonction hydrogénante est choisie dans le groupe formé par les combinaisons des éléments cobalt-molybdène, nickel-molybdène, ou nickel-cobalt-molybdène, ou nickel-molybdène-tungstène.

Dans le cas où une activité importante en hydrodésulfuration, ou en hydrodéazotation et en hydrogénation des aromatiques est souhaitée, la fonction hydro-deshydrogénante est avantageusement assurée par l'association de nickel et de molybdène ; une association de nickel et de tungstène en présence de molybdène peut également être avantageuse. Dans le cas des charges de type distillats sous vide ou plus lourdes, des combinaisons de type cobalt-nickel-molybdène peuvent être avantageusement utilisées.

Les précurseurs de ces éléments, et en particulier du molybdène, du tungstène, du groupe VIII, qui peuvent être utilisés sont également bien connus de l'homme du métier, ainsi que leur mode d'introduction. On se reportera par exemple à la demande de brevet WO-2011/80407.

La quantité de précurseur(s) d' (des) élément(s) du groupe VIB est avantageusement comprise entre 5 et 40 % poids d'oxydes du groupe VIB par rapport au poids du précurseur catalytique séché, calciné ou régénéré, autrement dit, perte au feu déduite (à 550°C, pression atmosphérique), de préférence entre 8 et 35 % poids et de manière très préférée entre 10 et 30 % poids.

La quantité de précurseur(s) d' (des) élément(s) du groupe VIII est avantageusement comprise entre 1 et 10 % poids d'oxydes du groupe VIII par rapport au poids du précurseur catalytique séché, calciné ou régénéré, autrement dit, perte au feu déduite, de préférence entre 1,5 et 9 %poids et de manière très préférée, entre 2 et 8 % poids.

Les dopants

Le phosphore est toujours présent dans le catalyseur obtenu. Il a généralement été introduit lors de l'imprégnation du support avec l'un au moins des éléments de la fonction hydro-déshydrogénante, donc il est présent sur le précurseur catalytique . Il peut également être introduit lors de l'imprégnation avec le(s) acide(s) carboxylique(s) lors de l'étape 1) du procédé et/ou lors de l'imprégnation avec le succinate (étape 2) du procédé.

Dans le cas où le procédé selon l'invention est utilisé pour la préparation de catalyseur frais séché ou calciné, le phosphore est de préférence introduit en totalité sur le précurseur catalytique, et de préférence par imprégnation.

Un autre dopant peut également être présent qui est de préférence choisi parmi le bore et le fluor pris seul ou en mélange. Le dopant est un élément ajouté, qui en lui-même ne présente aucun caractère catalytique mais qui accroît l'activité catalytique du (des) métal (métaux).

Les sources de bore ou de fluor sont connues ainsi que leur mode d'introduction et on se reportera par exemple à la demande de brevet WO-2011/80407.

La source de phosphore préférée est l'acide orthophosphorique $H_3PO_4$, mais ses sels et esters comme phosphates d'ammonium conviennent également. Le phosphore peut également être introduit en même temps que le(s) élément(s) du groupe VIB sous la forme d'hétéropolyanions de Keggin, Keggin lacunaire, Keggin substitué ou de type Strandberg.

Le dopant est introduit dans le précurseur catalytique dans une quantité d'oxyde dudit dopant par rapport au poids du catalyseur , perte au feu déduite (à 550°C, pression atmosphérique) :

- comprise entre 0 et 40 % poids, de préférence de entre 0 et 30 % poids et de manière encore plus préférée entre 0 et 20 % poids, de préférence entre 0 et 15 % poids et de manière encore plus préférée entre 0 et 10 % poids lorsque ledit dopant est le bore; lorsque le bore est présent, de préférence la quantité minimum est de 0,1% ou de manière préférée 0,5% poids.

- comprise entre 0,1 (ou 0,5%) à 20 % poids, de préférence entre 0,1 (ou 0,5%) et 15 % poids et de manière encore plus préférée entre 0,1 (ou 0,5%) et 10 % poids, lorsque ledit dopant est le phosphore.

- comprise entre 0 et 20 % poids, de préférence entre 0 et 15 % poids et de manière encore plus préférée entre 0 et 10 % poids, lorsque ledit dopant est le fluor ; lorsque le fluor est présent, de préférence la quantité minimum est de 0,1% ou 0,5% poids.

Avantageusement, le phosphore est introduit, en totalité ou en partie, en mélange avec le(s) précurseur(s) de la fonction hydro-déshydrogénante, sur le support amorphe mis en forme, de préférence des extrudés l'alumine ou de silice-alumine, par une imprégnation à sec dudit support amorphe à l'aide d'une solution contenant les sels précurseurs des métaux et le(s) précurseur(s) du (des) dopant(s).

De manière encore plus préférée, le "précurseur catalytique" du procédé selon l'invention, et en particulier le précurseur séché ou calciné frais, est préparé avec une solution d'imprégnation contenant au moins un précurseur de chaque élément de la fonction hydro-déshydrogénante, en présence d'un précurseur de phosphore, le support étant constitué d'alumine et/ou de silice-alumine et/ou de zéolithe.

Il peut être ajouté un additif pour augmenter l'activité catalytique. Par exemple, le précurseur catalytique frais (non usé) peut également avoir été préparé selon le procédé de préparation décrit dans WO-2011/80407. Il est également envisageable de remplacer le couple succinate/acide par le succinate seul ou par tout autre composé organique connu pour améliorer l'activité du catalyseur ; ces composés sont connus; ce sont par exemple des alcools C1-C10 comprenant au moins 2 fonctions alcool, des acides carboxyliques seuls tels que l'acide citrique, des molécules complexantes... On appellera ces précurseurs des "précurseurs catalytiques additivés". En général, ils sont traités selon le procédé de la présente invention, après avoir été usés et régénérés (c'est-à-dire à l'état de précurseur catalytique régénéré).

Les traitements thermiques pouvant être subis par le précurseur catalytique

Le précurseur catalytique séché a été obtenu par séchage à une température inférieure à 200°C et le plus souvent inférieure à 180°C. Elle est par exemple comprise entre 50 et 180°C, de manière préférée entre 60 et 150°C ou encore entre 65 et 145°C et de manière très préférée entre 70 et 140°C ou encore entre 75 et 130°C.

Le précurseur catalytique séché a été éventuellement calciné à une température d'au moins 350°C. La température de calcination est inférieure à 600°C et le plus souvent inférieure à 550°C, par exemple de 350 à 550°C, et de préférence entre 400 et 520°C, ou de façon préférée entre 420 et 520°C ou entre 450 et 520°C, des températures inférieures à 500°C sont souvent avantageuses.

Dans un autre mode, le catalyseur usé est régénéré La régénération est un traitement thermique en présence d'oxygène, pur ou dilué. Cette étape à pour but d'éliminer au moins une partie du coke présent sur le catalyseur par combustion. Il n'y a pas de traitement chimique lors de cette étape. Le traitement de régénération peut être effectué à un température comprise entre 350 et 550°C, et généralement entre 450 et 520°C, ou entre 420 et 520°C, ou entre 400 et 520°C. Elle est réalisée de préférence entre 420 et 500°C, ou entre 450 et 520°C selon la nature du carbone à brûler. L'homme du métier optimise la température nécessaire au brûlage du coke (ou de ses précurseurs) tout en évitant ou minimisant le frittage du catalyseur.

Durant cette étape un contrôle de la température est nécessaire de manière à permettre la combustion du coke mais à ne pas dépasser 550°C sur le catalyseur, y compris localement. Le dépassement de la température de 550°C pourrait par exemple avoir comme conséquence d'endommager sa porosité. Ce contrôle est connu de l'homme du métier. La température au sein du lit durant cette phase de régénération peut être contrôlée par toute technique connue de l'Homme du métier, comme par exemple la disposition de thermocouples dans la masse du catalyseur.

Lorsque cette étape est effectuée avec un mélange comprenant de l'oxygène, le diluant peut être choisi parmi l'azote ou tout autre gaz inerte. La teneur en oxygène peut être fixe tout au long du traitement ou varier au cours du processus de régénération. Par exemple, la température pourra évoluer au cours du traitement selon plusieurs phases, les températures pourront varier de l'ambiante à la température finale de combustion du coke, toujours inférieure à 550°C. La durée de cette étape de régénération dépendra de la quantité de catalyseur à traiter et de la nature et de la quantité du coke présent. Cette durée peut varier en pratique de 0,1 heure à quelques jours. Le plus souvent, elle est comprise entre 1 heure et 20 heures.

## Les étapes 1 et 2

L'étape 1)

Conformément à l'étape 1) du procédé selon l'invention, ledit précurseur catalytique séché ou calciné ou régénéré est imprégné par une solution d'imprégnation comprenant au moins un acide carboxylique, éventuellement au moins un composé de phosphore et éventuellement de l'acide acétique.

Lesdits composés sont avantageusement introduits dans la solution d'imprégnation de l'étape 1) du procédé selon l'invention dans une quantité correspondant (par rapport au précurseur catalytique):

- à un rapport molaire d'acide carboxylique par élément(s) du groupe VIB du précurseur catalytique compris entre 0,05 à 5,0 mole/mole, de préférence compris entre 0,1 à 4,0 mole/mole, de manière préférée compris entre 0,2 et 3,0 mole/mole et de manière très préférée, compris entre 0,5 et 2,0 mole/mole,

- à un rapport molaire de phosphore par élément(s) du groupe VIB du précurseur catalytique compris entre 0 à 1,0 mole/mole, de préférence compris entre 0 à 0,8 mole/mole, de manière préférée compris entre 0 et 0,6 mole/mole et de manière très préférée, compris entre 0 et 0,5 mole/mole,

- et, lorsque l'acide acétique est présent, à un rapport molaire d'acide acétique par élément(s) du groupe VIB du précurseur catalytique compris entre 0,1 à 6,0 mole/mole, de préférence compris entre 0,5 à 5,0 mole/mole, de manière préférée compris entre 1,0 et 4 mole/mole et de manière très préférée, compris entre 1,5 et 2,5 mole/mole,

- le rapport molaire d'acide carboxylique + acide acétique par élément(s) du groupe VIB du précurseur catalytique étant compris entre 0,15 à 11,0 mole/mole.

Ladite solution d'imprégnation peut avantageusement être déposée en une ou plusieurs étapes soit par imprégnation en slurry, soit par imprégnation en excès, soit par imprégnation à sec, soit par tout autre moyen connus de l'homme du métier. De préférence, c'est une seule étape d'imprégnation à sec.

La solution d'imprégnation comprend au moins un acide carboxylique autre que l'acide acétique, éventuellement au moins un composé de phosphore et éventuellement de l'acide acétique. De préférence, l'acide carboxylique est l'acide citrique.

La solution d'imprégnation peut contenir un solvant polaire. Il est avantageusement choisi dans le groupe formé par le méthanol, l'éthanol, l'eau, le phénol, le cyclohexanol, pris seuls ou en mélange. Il peut également être choisi dans le groupe formé par le carbonate de propylène, le DMSO (diméthylsulfoxyde) ou le sulfolane, pris seul ou en mélange. De manière préférée, on utilise un solvant protique polaire. Une liste des solvants polaires usuels ainsi que leur constante diélectrique peut être trouvée dans le livre "Solvents and Solvent Effects in Organic Chemistry, C. Reichardt, Wiley-VCH, 3eme édition, 2003, pages 472-474).

De préférence, le solvant est l'eau et/ou l'éthanol, et de préférence la solution d'imprégnation est une solution aqueuse.

Les acides carboxyliques utilisables dans la présente invention contiennent de 1 à 20 atomes de carbone avec au moins une fonction COOH, et de préférence au moins 2 fonctions COOH. Il est possible d'utiliser des acides ayant jusqu'à 3 , 4 voire et jusqu'à six fonctions COOH. Ces acides peuvent également contenir d'autres hétéroatomes (soufre, azote) et les fonctions chimique associées à ceux-ci. Dans un mode de réalisation préféré on utilisera des acides carboxyliques ne contenant pas d'autres hétéroétomes. De tels acides peuvent alors être choisis dans la liste suivante, non exhaustive : acide formique, acide maléique, acide propionique, acide butyrique acide valérique, acide caproïque, acide énanthique, acide caprylique acide pélargonique, acide caprique, acide undécylique, acide laurique, acide tridécylique, acide benzoïque, acide salicylique, acide malonique, acide succinique, acide glutarique, acide adipique, acide pimélique, acide subérique, acide azélaïque, acide phtalique, acide isophtalique, acide glycolique, acide lactique, acide malique, acide tartrique, acide citrique. Dans cette liste, on préfèrera les acides comprenant deux fonctions carboxyliques ou plus. En particulier, les acide citrique, tartrique, acide malique, acide malonique, acide glutarique, acide succinique sont préférés. L'acide citrique est encore plus préféré.

De préférence, la solution d'imprégnation contient uniquement l'acide citrique et éventuellement l'acide acétique, ainsi que de l'eau et/ou de l'éthanol. De préférence, c'est une solution aqueuse d'acide citrique ..

La solution d'imprégnation peut contenir un solvant non protique connu de l'homme du métier notamment le toluène, le xylène.

L'homme du métier choisira le(s) solvant(s) en fonction de la compatibilité avec les composantes de la solution.

Les conditions de la maturation sont décrites plus loin.

Le précurseur catalytique ainsi imprégné est soumis à une étape de séchage. Le but de cette étape est d'enlever tout ou partie de l'éventuel solvant ayant permis l'introduction de l'acide carboxylique, ceci avant l'étape 2).

Cette étape de séchage est avantageusement effectuée par toute technique connue de l'homme du métier. Elle est avantageusement effectuée à pression atmosphérique ou à pression réduite. De manière préférée, cette étape est réalisée à pression atmosphérique.

Elle est effectuée à une température inférieure à 200°C, généralement comprise entre 50°C et 200°C, de préférence comprise entre 60 et 190°C. De manière très préférée, elle est inférieure à 180°C, de préférence comprise entre 60 et 180°C, et avantageusement entre 80 et 180°C ou 100 et 180°C. Généralement, on opère dans ces gammes de températures et sans traitement thermique ultérieur à une température de 200°C ou plus.

Elle est avantageusement effectuée en four tunnel, en lit fluidisé, en lit fluidisé vibré, en lit fluidisé à échangeurs, en lit traversé ou toute technologie permettant le séchage . De manière préférée, le gaz utilisé est soit l'air, soit un gaz inerte comme l'argon ou l'azote. De manière très préférée, le séchage est réalisé sous azote.

De préférence, cette étape a une durée comprise entre 30 min et 4 h et de préférence entre 45 min et 3 h.

Le séchage permet l'élimination d'une partie ou de la totalité du solvant, ce qui libère du volume poreux et le rend disponible pour l'étape 2. De préférence, la température de séchage est supérieure à la température d'ébullition ou de décomposition de l'acide carboxylique. Ce ci permet d'éliminer l'acide en partie ou en totalité. Dans certains cas, et notamment dans le cas où un acide lourd est utilisé, comme cela a été précédemment décrit, il peut être avantageux de prolonger le séchage par un traitement thermique à une température inférieure à 350°C.

L'étape 2)

Le précurseur catalytique séché ou calciné ou régénéré qui a été imprégné et séché ou traité thermiquement selon l'étape 1) est maintenant imprégné par une solution d'imprégnation comprenant au moins un succinate de dialkyle C1-C4 (et en particulier du succinate de diméthyle), et éventuellement au moins un composé de phosphore, et éventuellement de l'acide acétique.

Lesdits composés sont avantageusement introduits dans la solution d'imprégnation dans une quantité correspondant (par rapport au précurseur catalytique) :

- à un rapport molaire de succinate de dialkyle (par ex diméthyle) par élément(s) du groupe VIB du précurseur catalytique compris entre 0,1 à 2,0 mole/mole, de préférence compris entre 0,2 à 1,8 mole/mole, de manière préférée compris entre 0,2 et 1,5 mole/mole et de manière très préférée, compris entre 0,5 et 1,0 mole/mole,

- à un rapport molaire de phosphore par élément(s) du groupe VIB du précurseur catalytique compris entre 0 à 1,0 mole/mole, de préférence compris entre 0 à 0,8 mole/mole, de manière préférée compris entre 0 et 0,6 mole/mole et de manière très préférée, compris entre 0 et 0,5 mole/mole,

- et , lorsque l'acide acétique est présent, à un rapport molaire d'acide acétique par élément(s) du groupe VIB du précurseur catalytique compris entre 0,1 à 6 mole/mole, de préférence compris entre 0,3 à 5 mole/mole, de manière préférée compris entre 0,5 et 3 mole/mole et de manière très préférée, compris entre 0,7 et 2,0 mole/mole,

Ladite solution d'imprégnation peut avantageusement être déposée en une ou plusieurs étapes soit par imprégnation en slurry, soit par imprégnation en excès, soit par imprégnation à sec, soit par tout autre moyen connus de l'homme du métier. De préférence, c'est une seule étape d'imprégnation à sec.

Une solution d'imprégnation préférée comprend au moins un succinate de dialkyle et au moins un composé de phosphore, et éventuellement de l'acide acétique. Une autre solution d'imprégnation préférée comprend au moins un succinate de dialkyle , de l'acide acétique et éventuellement au moins un composé de phosphore. De préférence, la solution contient de l'acide acétique. De préférence, la solution contient le succinate de diméthyle et l'acide acétique.

La solution d'imprégnation peut contenir un solvant polaire. Il est avantageusement choisi dans le groupe formé par le méthanol, l'éthanol, l'eau, le phénol, le cyclohexanol, pris seuls ou en mélange. Il peut également être choisi dans le groupe formé par le carbonate de propylène, le DMSO (diméthylsulfoxyde) ou le sulfolane, pris seul ou en mélange. De manière préférée, on utilise un solvant protique polaire. Une liste des solvants polaires usuels ainsi que leur constante diélectrique peut être trouvée dans le livre "Solvents and Solvent Effects in Organic Chemistry, C. Reichardt, Wiley-VCH, 3eme édition, 2003, pages 472-474).

De préférence, le solvant est l'eau et/ou l'éthanol, et de préférence la solution d'imprégnation est une solution aqueuse.

Le succinate de dialkyle utilisé est de préférence compris dans le groupe composé du succinate de diméthyle, du succinate de diéthyle, du succinate de dipropyle , du succinate de diisopropyle et du succinate de dibutyle. De manière préférée, le succinate de dialkyle C1-C4 utilisé est le succinate de diméthyle ou le succinate de diéthyle. De manière très préférée, le succinate de dialkyle C1-C4 utilisé est le succinate de diméthyle. Au moins un succinate de dialkyle C1-C4 est utilisé, de préférence un seul, et de préférence le succinate de diméthyle.

Les conditions de maturation

La maturation est une étape lors de laquelle la solution imprégnée est laissée en contact avec le précurseur catalytique. La maturation dans les deux étapes est avantageusement réalisée à pression atmosphérique. La température est généralement comprise entre 17°C et 60°C ou plus avantageusement entre 17°C et 50°C. Généralement, la durée de maturation est généralement supérieure à 10 min, de manière préférée comprise entre 10 min et 48 h, de manière encore plus préférée entre 20 min et 24h et avantageusement comprise entre 30 min et 6 h. Une durée de 6 h est souvent suffisante. Des durées plus longues ne sont pas cependant pas à exclure.

Un moyen simple d'ajuster la durée de maturation de l'étape 2) est de caractériser la formation des hétéropolyanions de Keggin par spectroscopie Raman. De manière très préférée, pour augmenter la productivité sans modifier la quantité d'hétéropolyanions reformés, la durée de la maturation, à température ambiante est comprise entre 30 min et 6 h. Cette durée peut être réduite à condition de chauffer le précurseur imprégné à une température d'au plus 60°C.

De la même manière, un moyen d'ajuster la durée de la première étape de maturation, est de caractériser la disparition des espèces cristallines réfractaires à la sulfuration, par exemple le $CoMoO_4$. Ceci peut être fait par DRX ou de manière encore plus fine par spectroscopie Raman (doublet de raies à 939 et 948 cm$^{-1}$).

Le séchage final

Le produit ainsi imprégné selon l'étape 2) est soumis à une étape de séchage. Le but de cette étape est d'obtenir un catalyseur transportable, stockable, et manipulable, en particulier pour le chargement de l'unité d'hydrotraitement. Il s'agit d'enlever tout ou partie de l'éventuel solvant ayant permis l'introduction du succinate de dialkyle C1-C4 (en particulier de diméthyle). Dans tous les cas, il s'agit de donner un aspect sec au catalyseur, afin d'éviter que les extrudés ne se collent les uns aux autres durant les étapes de transport, de stockage, de manipulation ou de chargement.

Cette étape de séchage est avantageusement effectuée par toute technique connue de l'homme du métier. Elle est avantageusement effectuée à pression atmosphérique ou à pression réduite. De manière préférée, cette étape est réalisée à pression atmosphérique.

.Elle est effectuée à une température inférieure à 200°C, généralement comprise entre 50°C et 200°C, de préférence comprise entre 60 et 190°C. De manière très préférée, elle est inférieure à 180°C, de préférence comprise entre 50°C (ou 60°C) et 180°C (ou 160°C), et avantageusement entre 80 et 180°C ou 50 et 160°C. On opère sans traitement thermique ultérieur à une température de 200°C ou plus.

Elle est avantageusement effectuée en four tunnel, en lit fluidisé, en lit fluidisé vibré, en lit fluidisé à échangeurs, en lit traversé ou toute technologie permettant le séchage. De manière préférée, le gaz utilisé est soit l'air, soit un gaz inerte comme l'argon ou l'azote. De manière très préférée, le séchage est réalisé sous azote.

De préférence, cette étape a une durée comprise entre 30 min et 4 h et de préférence entre 45 min et 3 h.

A l'issue de l'étape 2) du procédé selon l'invention, on obtient un catalyseur séché, qui n'est soumis à aucune étape de calcination ultérieure ou de traitement thermique ultérieur à une température de 200°C ou plus.

Le catalyseur obtenu à l'issue de l'étape 2) présente un spectre Raman comprenant les bandes les plus intenses à 990 et/ou 974 cm$^{-1}$ (hétéropolyanions de type Keggin), les bandes correspondant au succinate (pour le succinate de diméthyle la bande la plus intense est à 853 cm$^{-1}$).

### La sulfuration

Avant son utilisation, le catalyseur obtenu à l'issue de l'étape 2) est transformé en un catalyseur sulfuré afin de former sa phase active. Cette phase d'activation ou de sulfuration s'effectue par les méthodes bien connues de l'homme de l'art, et avantageusement sous une atmosphère sulfo-réductrice en présence d'hydrogène et d'hydrogène sulfuré.

A l'issue de l'étape 2) le catalyseur séché obtenu est donc avantageusement soumis à une étape de sulfuration, sans étape de calcination intermédiaire. Il est obtenu un catalyseur sulfuré, selon l'invention.

Ledit catalyseur séché est avantageusement sulfuré de manière *ex situ* ou *in situ.* Les agents sulfurants sont le gaz $H_2S$ ou tout autre composé contenant du soufre utilisé pour l'activation des charges hydrocarbures en vue de sulfurer le catalyseur. Lesdits composés contenant du soufre sont avantageusement choisis parmi les alkyldisulfures tels que par exemple le disulfure de diméthyle (DMDS), les alkylsulfures, tel que par exemple le sulfure de diméthyle, le n-butylmercaptan, les composés polysulfures de type tertiononylpolysulfure tels que par exemple le TPS-37 ou le TPS-54 commercialisés par la société ARKEMA, ou tout autre composé connus de l'homme du métier permettant d'obtenir une bonne sulfuration du catalyseur. De manière préféré le catalyseur est sulfuré *in situ* en présence d'un agent sulfurant et d'une charge hydrocarbonée. De manière très préférée le catalyseur est sulfurée *in situ* en présence d'une charge hydrocarbonée additivée de dissulfure de diméthyle.

### PROCEDE D'HYDROTRAITEMENT ET/OU D'HYDROCONVERSION utilisant le catalyseur obtenu à l'issue de l'étape 2 et de préférence activé:

Enfin, un autre objet de l'invention est un procédé d'hydrotraitement de charges hydrocarbonées utilisant le catalyseur préparé selon l'invention. De tels procédés sont, par exemple, les procédés d'hydrodésulfuration, d'hydrodéazotation, d'hydrodémétallation, d'hydrogénation des aromatiques, procédés qu'on inclura sous la dénomination "hydrotraitement" . Un autre objet de l'invention est un procédé et d'hydroconversion de charges hydrocarbonées utilisant le catalyseur préparé selon l'invention.

Les catalyseurs séchés obtenus par le procédé selon l'invention et ayant de préférence préalablement subi une de sulfuration sont avantageusement utilisés pour les réactions d'hydrotraitement de charges hydrocarbonées telles que les coupes pétrolières, les coupes issues de la conversion du charbon ou les hydrocarbures produits à partir du gaz naturel .Ces catalyseurs sont par exemple avantageusement utilisés lors du pré-traitement des charges de craquage catalytique ou l'hydrodésulfuration des résidus ou l'hydrodésulfuration poussée des gazoles (ULSD Ultra Low Sulfur Diesel)

Les catalyseurs obtenus par le procédé selon l'invention et ayant de préférence préalablement subi une étape de sulfuration présentent une activité améliorée par rapport aux catalyseurs de l'art antérieur.

Les charges employées dans les procédés d'hydrotraitement sont par exemple des essences, des gas-oils, des gas-oils sous vide, des résidus atmosphériques, des résidus sous vide, des distillats atmosphériques, des distillats sous vide, des fuels lourds, des huiles, des cires et des paraffines, des huiles usagées, des résidus ou des bruts désasphaltés, des charges provenant des procédés de conversions thermiques ou catalytiques, prises seules ou en mélanges. Les charges qui sont traitées, et en particulier celles citées ci-dessus, contiennent généralement des hétéroatomes tels que le soufre, l'oxygène et l'azote et, pour les charges lourdes, elles contiennent le plus souvent également des métaux.

Les conditions opératoires utilisées dans les procédés mettant en oeuvre les réactions d'hydrotraitement de charges hydrocarbonées décrites ci-dessus sont généralement les suivantes : le température est avantageusement comprise entre 180 et 450 °C, et de préférence entre 250 et 440 °C, la pression est avantageusement comprise entre 0,5 et 30 MPa, et de préférence entre 1 et 18 MPa, la vitesse volumique horaire est avantageusement comprise entre 0,1 et 20 $h^{-1}$ et de préférence entre 0,2 et 5 $h^{-1}$, et le rapport hydrogène/charge exprimé en volume d'hydrogène, mesuré dans les conditions normales de température et pression, par volume de charge liquide est avantageusement compris entre 50 L/L à 2000 L/L.

Le procédé d'hydroconversion opère en présence d'hydrogène, à une température supérieure à 200°C, de préférence comprise entre 250 et 480°C, de manière préférée entre 320 et 450°C, de manière très préférée entre 330 et 435°C, sous une pression supérieure à 1 MPa, de préférence entre 2 et 25 MPa, de manière préférée entre 3 et 20 MPa, à la vitesse spatiale comprise entre 0,1 et 20 h-1, de préférence 0,1 et 6 h-1, de manière préférée entre 0,2 et 3 h-1, et la quantité d'hydrogène introduite est telle que le rapport volumique litre d'hydrogène/litre d'hydrocarbure est compris entre 80 et 5000 L/L et le plus souvent entre 100 et 3000 L/L. Ces conditions opératoires permettent généralement d'atteindre des conversions par passe, en produits ayant des points d'ébullition inférieurs à 300°C, et mieux inférieurs à 340°C, et encore mieux inférieurs à 370°C, d'au moins 50% pds et de manière encore plus préférée comprises entre 20 et 100% mais le plus généralement entre 60-95%pds.

Des charges très variées peuvent être traitées par les procédés selon l'invention décrits ci-dessus. Elles contiennent avantageusement au moins 20% volume et de préférence au moins 80% volume de composés bouillant au-dessus de 340°C.

La charge est avantageusement choisie parmi les LCO (Light Cycle Oil: gazoles légers issus d'une unité de craquage catalytique), les distillats atmosphériques, les distillats sous vide tels que par exemple issus de la distillation directe du brut ou d'unités de conversion telles que le FCC, le coker ou la viscoréduction, les charges provenant d'unités d'extraction d'aromatiques des bases d'huile lubrifiante ou issues du déparaffinage au solvant des bases d'huile lubrifiante, les distillats provenant de procédés de désulfuration ou d'hydroconversion en lit fixe, en lit bouillonnant ou en slurry de RAT (résidus atmosphériques) et/ou de RSV (résidus sous vide) et/ou d'huiles désasphaltées, et les huiles désasphaltées, prises seules ou en mélange. La liste ci-dessus n'est pas limitative.

## EXEMPLES

Les exemples qui suivent démontrent le gain d'activité important sur les catalyseurs préparés selon le procédé selon l'invention par rapport aux catalyseurs de l'art antérieur et précisent l'invention sans toutefois en limiter la portée.

Exemple 1 : préparation des catalyseurs A1, A2 et A3 (non conformes) et A4 (conforme)

Une matrice composée de boehmite tabulaire ultrafine ou gel d'alumine, commercialisée par la société Condéa Chemie GmbH a été utilisée. Ce gel a été mélangé à une solution aqueuse contenant de l'acide nitrique à 66 % (7 % en poids d'acide par gramme de gel sec), puis malaxé pendant 15 minutes. A l'issue de ce malaxage, la pâte obtenue est passée à travers une filière ayant des orifices cylindriques de diamètre égal à 1,6 mm. Les extrudés sont ensuite séchés pendant une nuit à 120 °C, puis calcinés à 600 °C pendant 2 heures sous air humide contenant 50 g d'eau par kg d'air sec. On obtient ainsi des extrudés de support uniquement composé d'alumine gamma cubique de faible cristallinité.

Sur le support d'alumine décrit précédemment et qui se présente sous la forme extrudée on ajoute du cobalt, du molybdène et du phosphore. La solution d'imprégnation est préparée par dissolution à chaud de l'oxyde de molybdène (24,34 g) et d'hydroxyde de cobalt (5,34 g) dans la solution d'acide phosphorique (7,47 g) en solution aqueuse. Après imprégnation à sec, les extrudés sont laissés à maturer à température ambiante (20°C) en atmosphère saturée en eau pendant 12 h, puis ils sont séchés une nuit à 90°C. On obtient le précurseur catalytique séché A1. Une fraction du catalyseur séché est alors calcinée à 450°C pendant 2 heures. On obtient le catalyseur calciné A2. La composition finale des catalyseurs A1 et A2 exprimée sous forme d'oxydes est alors la suivante : $MoO_3$ = 22,5 $\pm$ 0,2 (% en poids), CoO = 4,1 $\pm$ 0,1 (% en poids) et $P_2O_5$ = 4,0 $\pm$ 0,1 (% en poids).

Le catalyseur A3 est préparé par imprégnation à sec du précurseur séché A1 avec une solution comprenant du diméthyle succinate et de l'acide acétique dilués dans l'eau. Les teneurs visées en diméthyle succinate (DMSU) et en Acide acétique (AA) sont respectivement de 27 % poids et 18 % poids (soit AA/Mo=1,9 mol/mol et DMSU/Mo=1,2 mol/mol). Après une durée de maturation de 3 heures en vase clos à température ambiante, le catalyseur est une nouvelle fois séché sous flux d'azote (1 NL/g/g) durant 1 heure en lit traversé à 140°C.

Le catalyseur A4 est préparé par imprégnation à sec du précurseur calciné A2 avec une solution d'acide citrique dilué dans l'eau. Les teneurs visées en acide citrique (AC) sont 10 % poids (soit AC/Mo=0,35 mol/mol). Après une durée de maturation de 12 heures en vase clos à température ambiante, le catalyseur est séché sous flux d'azote (1 NL/g/g) durant 2 heures dans un four de type lit traversé, à 180°C. Le catalyseur est ensuite imprégné à sec avec une solution comprenant du diméthyle succinate et de l'acide acétique dilués dans l'eau. Les teneurs visées en diméthyle succinate (DMSU) et en Acide acétique (AA) sont respectivement de 18 % poids et 17 % poids (soit AA/Mo=1,8 mol/mol et DMSU/Mo=0,8 mol/mol). Après une durée de maturation de 3 heures en vase clos à température ambiante, le catalyseur est une nouvelle fois séché sous flux d'azote (1 NL/g/g) durant 1 heure en lit traversé à 140°C.

Exemple 2 : préparation du catalyseur régénéré B31

Le catalyseur A3 est chargé dans une unité lit traversé et sulfuré par un gazole de distillation directe additivé de 2% poids de disulfure de diméthyle. Un test d'HDS d'un mélange de gazole de distillation directe et d'un gazole en provenance du craquage catalytique est alors conduit pendant 600 h. Après test, le catalyseur usé est déchargé, recueilli et lavé au toluène à reflux durant 12 heures puis séparé en deux lots. Le premier lot est régénéré en four de combustion contrôlée en introduisant pour chaque palier de température des quantités croissantes d'oxygène, ce qui permet de limiter l'exothermie lié à la combustion du coke. Le palier final de régénération est de 480°C. Le catalyseur ainsi régénéré est analysé par DRX. On note la présence d'une raie à 26° caractéristique de la présence de $CoMoO_4$ cristallisé. En outre, ce catalyseur qui sera désormais noté B31 possède une couleur bleu vif très prononcée.

Exemple 3 : préparation des catalyseurs C31 et C31bis (non conformes) à partir du précurseur régénéré B31 - réalisation avec l'acide citrique seul

Les catalyseurs C31 et C31bis sont préparés par imprégnation à sec du catalyseur régénéré B31 avec une solution d'acide citrique dilué dans l'eau. Les teneurs visées en acide citrique (AC) sont 10 % poids (soit AC/Mo=0,35 mol/mol). Après une durée de maturation de 12 heures en vase clos à température ambiante, le catalyseur est partagé en deux lots : le premier est séché sous flux d'azote (1 NL/g/g) durant 2 heures dans un four de type lit traversé, à 180°C, conduisant au catalyseur C31. Le second lot est séché à l'identique, mais avec une consigne de température de 140°C, conduisant au catalyseur C31bis.

Exemple 4 : préparation d'un catalyseur D31 et D31bis (conformes) à partir de précurseur régénéré - réalisation avec le DMSU

Le catalyseur D31 est préparé par imprégnation à sec du catalyseur régénéré C31 avec une solution comprenant du succinate de diméthyle (DMSU) pur. Cela revient à viser 32 % poids de succinate de diméthyle sur le catalyseur final (soit DMSU/Mo=1,4 mol/mol). Après une durée de maturation de 3 heures en vase clos à température ambiante, le catalyseur est une nouvelle fois séché sous flux d'azote (1 NL/g/g) durant 1 heure en lit traversé à 140°C.
Le catalyseur D31bis est préparé par imprégnation à sec du catalyseur régénéré C31bis avec une solution comprenant du succinate de diméthyle (DMSU) pur. Cela revient à viser 30 % poids de succinate de diméthyle sur le catalyseur final (soit DMSU/Mo=1,3 mol/mol). Après une durée de maturation de 3 heures en vase clos à température ambiante, le catalyseur est une nouvelle fois séché sous flux d'azote (1 NL/g/g) durant 1 heure en lit traversé à 140°C.
Les catalyseurs D31 et D31bis ont été analysés par spectroscopie Raman. Ils présentent tout deux notamment la bande principale de l'HPA de Keggin à 990 $cm^{-1}$.

Exemple 5 : préparation d'un catalyseur régénéré E31 et E31bis (conformes) - réalisation avec le DMSU et l'acide acétique

Le catalyseur E31 est préparé par imprégnation à sec du catalyseur régénéré C31 avec une solution avec une solution comprenant du diméthyle succinate et de l'acide acétique dilués dans l'eau. Les teneurs visées en diméthyle succinate (DMSU) et en Acide acétique (AA) sont respectivement de 18 % poids et 17 % poids (soit AA/Mo=1,8 mol/mol et DMSU/Mo=0,8 mol/mol). Après une durée de maturation de 3 heures en vase clos à température ambiante, le catalyseur est une nouvelle fois séché sous flux d'azote (1 NL/g/g) durant 1 heure en lit traversé à 140°C.
Le catalyseur E31bis est préparé par imprégnation à sec du catalyseur régénéré C31bis avec une solution avec la même solution que pour E31, celle-ci comprenant du diméthyle succinate et de l'acide acétique dilués dans l'eau. Les teneurs visées en diméthyle succinate (DMSU) et en Acide acétique (AA) sont respectivement de 17 % poids et 17 % poids (soit AA/Mo=1,8 mol/mol et DMSU/Mo=0,7 mol/mol). Après une durée de maturation de 3 heures en vase clos à température ambiante, le catalyseur est une nouvelle fois séché sous flux d'azote (1 NL/g/g) durant 1 heure en lit traversé à 140°C.
Les catalyseurs E31 et E31bis ont été analysés par spectroscopie Raman. Ils présentent notamment la bande principale de l'HPA de Keggin à 990 cm$^{-1}$, mais aussi un épaulement à 850 cm$^{-1}$ caractéristique du diméthyle-succinate (fig.1).

Exemple 6 : préparation d'un catalyseur régénéré F31 (non conforme) - réalisation en une étape

Le catalyseur F31 est préparé par imprégnation à sec du catalyseur régénéré B31 avec une solution comprenant du diméthyle succinate, de l'acide citrique et de l'acide acétique dilués dans l'eau. Les teneurs visées en acide citrique (AC), en diméthyle succinate (DMSU) et en acide acétique (AA) sont respectivement de 10 % poids, 18 % poids et 17 % poids (soit AC/Mo=0,3 mol/mol, DMSU/Mo=0,8 mol/mol et AA/Mo=1,8 mol/mol). Après une durée de maturation de 12 heures en vase clos à température ambiante, le catalyseur est séché sous flux d'azote (1 NL/g/g) durant 2 heure en lit traversé à 160°C.

Exemple 7 : Test comparatif des catalyseurs A1, A2, A3, A4, B31, C31, C31bis, D31, D31bis, E31, E31bis, F31, en hydrogénation du toluène dans le cyclohexane sous pression et en présence d'hydrogène sulfuré.

Les catalyseurs précédemment décrits, sont sulfurés in situ en dynamique dans le réacteur tubulaire à lit fixe traversé d'une unité pilote de type Microcat (constructeur : société Vinci), les fluides circulant de haut en bas. Les mesures d'activité hydrogénante sont effectuées immédiatement après la sulfuration sous pression et sans remise à l'air avec la charge d'hydrocarbures qui a servi à sulfurer les catalyseurs.

La charge de sulfuration et de test est composée de 5,8 % de diméthyldisulfure (DMDS), 20 % de toluène et 74,2 % de cyclohexane (en poids).

La sulfuration est effectuée dès température ambiante jusqu'à 350°C, avec une rampe de température de 2°C/min, une VVH = 4h$^{-1}$ et H$_2$/HC = 450 NL/L . Le test catalytique est effectué à 350°C à VVH = 2h$^{-1}$ et H$_2$/HC équivalent à celui de la sulfuration, avec prélèvement minimum de 4 recettes qui sont analysées par chromatographie en phase gazeuse.

On mesure ainsi les activités catalytiques stabilisées de volumes égaux de catalyseurs dans la réaction d'hydrogénation du toluène.

Les conditions opératoires détaillées dans lesquelles sont réalisées les mesures d'activité sont les suivantes :

| | |
|---|---|
| - Pression totale : | 6,0 MPa |
| - Pression de toluène : | 0,37 MPa |
| - Pression de cyclohexane : | 1,42 MPa |
| - Pression de méthane | 0,22 MPa |
| - Pression d'hydrogène : | 3,68 MPa |
| - Pression de H$_2$S : | 0,22 MPa |
| - Volume de catalyseur : | 4 cm$^3$ (extrudés de longueur comprise entre 2 et 4 mm) |
| - Vitesse spatiale horaire : | 2h$^{-1}$ |
| - Température de sulfuration et de test : | 350 °C |

Des prélèvements de l'effluent liquide sont analysés par chromatographie en phase gazeuse. La détermination des concentrations molaires en toluène non converti (T) et des concentrations ses produits d'hydrogénation (le méthylcyclohexane (MCC6), l'éthylcyclopentane (EtCC5) et les diméthylcyclopentanes (DMCC5)) permettent de calculer un taux d'hydrogénation de toluène X$_{HYD}$ défini par :

$$X_{HYD}(\%) = 100 \times \frac{MCC6 + EtCC5 + DMCC5}{T + MCC6 + EtCC5 + DMCC5}$$

La réaction d'hydrogénation du toluène étant d'ordre 1 dans les conditions de test mises en œuvre et le réacteur se comportant comme un réacteur piston idéal, on calcule l'activité hydrogénante A$_{HYD}$ des catalyseurs en appliquant la formule :

$$A_{HYD} = \ln\left(\frac{100}{100 - X_{HYD}}\right)$$

Le Tableau 1 compare les activités hydrogénantes relatives c'est-à-dire égales au rapport de l'activité du catalyseur sur l'activité du catalyseur B2 (non conforme) pris comme référence (activité 100 %) pour tous les catalyseurs préparés ici.

Les résultats des tests sont reportés dans le Tableau 1.

Le Tableau 1 montre que les catalyseurs additivés D31 et E31 (conformes) préparés par ajout de respectivement 32 % poids de Diméthyle succinate (DMSU) et 18% poids de Diméthyle succinate (DMSU) plus 10 %poids d'acide acétique au catalyseur C31 lui même préparé par ajout de 10%poids d'acide citrique (AC) au catalyseur B1 ont une activité améliorée par rapport au catalyseur de départ de respectivement 52 et 62%.

Comparativement les catalyseurs non conformes C31 (AC seul) ou F31 (imprégnation simultanée de AC, DMSU, AA) présentent des gains d'activité respectifs de 38 et 40 %.

Une diminution de la température du traitement thermique après l'étape d'imprégnation de l'acide citrique conduit à des catalyseurs D31bis et E31bis (conformes) d'activités similaires ou légèrement inférieures à respectivement D31 et E31. Ces catalyseurs présentent en particulier des activités supérieures de 10 et 17% au catalyseur imprégné en une seule étape F31.

L'imprégnation en deux étapes d'un acide carboxylique puis d'un succinate de dialkyle est aussi avantageuse sur un précurseur calciné, comme en témoignent les performances du catalyseur A4.

*Tableau 1 : Activités hydrogénantes relatives par rapport au catalyseur calciné A2 (non conforme)*

| Catalyseur | Type d'acide | Quantité d'acide (% poids par rapport au catalyseur final) | Type d'additif organique (étape2) | Quantité d'additif organique (% poids par rapport au catalyseur final) | Nombre d'imprégnation (acide et/ou additif) | $A_{HYD}$ relative par rapport à A2 (%) |
|---|---|---|---|---|---|---|
| A1 (séché, non conforme) | - | - | - | - | - | 95 |
| A2 (calciné, non conforme) | - | - | - | - | - | 100 |
| A3 (séché, non conforme) | AA | 18 | DMSU | 27 | 1 | 170 |
| **A4 (calciné, conforme)** | **AC (étape1) +AA (étape2)** | **AC=10 ; AA=17** | **DMSU** | **18** | **2** | **167** |
| B31 régénéré (non conforme) | - | 0 | - | 0 | - | 110 |
| C31 (non conforme) | AC | 10 | - | - | 1 | 148 |
| C31bis (non conforme) | AC | 10 | - | - | 1 | 151 |
| **D31 (conforme)** | AC | **10** | **DMSU** | **32** | **2** | **162** |
| **D31bis (conforme)** | AC | **10** | **DMSU** | **32** | **2** | **160** |
| **E31 (conforme)** | **AC (étape1) +AA (étape2)** | **AC=10 ; AA=17** | **DMSU** | **18** | **2** | **172** |

(suite)

| Catalyseur | Type d'acide | Quantité d'acide (% poids par rapport au catalyseur final) | Type d'additif organique (étape2) | Quantité d'additif organique (% poids par rapport au catalyseur final) | Nombre d'imprégnation (acide et/ou additif) | $A_{HYD}$ relative par rapport à A2 (%) |
|---|---|---|---|---|---|---|
| **E31bis (conforme)** | **AC (étape1) +AA (étape2)** | **AC=10 ; AA=17** | **DMSU** | **17** | **2** | **167** |
| F31 (non conforme) | AC+AA | **AC=10 ; AA=17** | DMSU | 18 | 1 | 150 |

Exemple 8 : Test comparatif des catalyseurs A2, A3, B31, C31, C31bis, E31, E31bis, F31, en HDS de Gazole.

Les catalyseurs précédemment décrits, sont sulfurés in situ en dynamique dans le réacteur tubulaire à lit fixe traversé d'une unité pilote de type Microcat (constructeur : société Vinci), les fluides circulant de haut en bas. Les mesures d'activité sont effectuées immédiatement après la sulfuration sous pression et sans remise à l'air avec un gazole de distillation directe.

La charge de sulfuration est composée de 2% de diméthyldisulfure (DMDS) ajoutés à un gazole de distillation directe. La sulfuration est effectuée dès température ambiante jusqu'à 350°C. Un palier de 12 heures à 350°C est observé pour cela.

Le test catalytique est effectué à trois températures : chronologiquement 330-335-340°C à VVH = 1h$^{-1}$ et $H_2$/HC de 450 Nl/l, avec prélèvement de recettes liquides toutes les 24 heures. Lorsque les teneurs en soufre dans les recettes sont stables, la température est changée. le test dure environ 400-450 heures au total.

Le soufre dans les effluents est analysé par FX. En traçant l'évolution de la teneur en soufre dans les effluents en fonction de la température, il est possible de mesurer les écarts relatifs de températures entre catalyseurs. On choisit ici de chiffrer l'écart de performances en degrés Celsius à 50 ppm : notée $T_{50}$-HDS. Le Tableau 2 compare les activités par rapport à l'activité du catalyseur A2 (non conforme) pris comme base. Les catalyseurs présentant, pour une teneur en soufre dans l'effluent de 50ppm, une température inférieure à celle du catalyseur de base, sont plus actifs.

On confirme l'intérêt de l'introduction séquentielle des acides carboxyliques et succinates. E31 et E3bis préparés selon l'invention permettent ainsi d'améliorer l'activité du catalyseur régénéré B31 de 5 et 6°C respectivement alors que l'imprégnation d'Acide citrique seul C31 (ou C31bis) ne permet de gagner que 2°C (ou 3°C) et que l'imprégnation simultanée du mélange DMSU, AC, AA ne permet de gagner que 3°C.

*Tableau 2 : Activités HDS relatives par rapport au catalyseur calciné A2 (non conforme)*

| Catalyseur | Type d'acide | Quantité d'acide (% poids par rapport au catalyseur final) | Type d'additif organique (étape2) | Quantité d'additif organique (% poids par rapport au catalyseur final) | Nombre d'imprégnation (acide et/ou additif) | $T_{50}$-HDS (°C) |
|---|---|---|---|---|---|---|
| A2 (calciné, non conforme) | - | - | - | - | - | base |
| A3 (séché, non conforme) | AA | 18 | DMSU | 27 | 1 | base - 6 |
| B31 régénéré (non conforme) | - | 0 | - | 0 | - | base - 1 |
| C31 (non conforme) | AC | 10 | - | - | 1 | base - 3 |

(suite)

| Catalyseur | Type d'acide | Quantité d'acide (% poids par rapport au catalyseur final) | Type d'additif organique (étape2) | Quantité d'additif organique (% poids par rapport au catalyseur final) | Nombre d'imprégnation (acide et/ou additif) | $T_{50}$-HDS (°C) |
|---|---|---|---|---|---|---|
| C31bis (non conforme) | AC | 10 | - | - | 1 | base - 4 |
| **E31 (conforme)** | **AC (étape1) +AA (étape2)** | **AC=10 ; AA=17** | **DMSU** | **18** | **2** | **base - 7** |
| **E31bis (conforme)** | **AC (étape1) +AA (étape2)** | **AC=10 ; AA=17** | **DMSU** | **17** | **2** | **base - 6** |
| F31 (non conforme) | AC+AA | 10+17 | DMSU | 18 | 1 | base - 4 |

**Revendications**

**1.** Procédé de préparation d'un catalyseur d'hydrotraitement et/ou d'hydroconversion à partir d'un précurseur catalytique comprenant un support à base d'alumine et/ou de silice-alumine et/ou de zéolite, et comprenant au moins un élément du groupe VIB et éventuellement au moins un élément du groupe VIII, ledit procédé comprenant l'imprégnation dudit précurseur par une solution d'un succinate de dialkyle C1-C4, **caractérisé en ce qu'**il comporte les étapes suivantes :

   1) imprégnation (étape 1) dudit précurseur séché , calciné ou régénéré, avec au moins une solution contenant au moins un acide carboxylique autre que l'acide acétique, puis maturation et séchage à une température inférieure à 200°C, éventuellement suivi d'un traitement thermique à une température inférieure à 350°C
   2) suivie d'une imprégnation (étape 2) avec une solution contenant au moins un succinate de dialkyle C1-C4 puis maturation et séchage à une température inférieure à 200°C, sans étape de calcination ultérieure

   et, le précurseur catalytique et/ou la solution de l'étape 1 et/ou la solution de l'étape 2 contient du phosphore.

**2.** Procédé selon la revendication 1 **caractérisé en ce que** le précurseur catalytique est un catalyseur qui a été régénéré.

**3.** Procédé selon l'une des revendications précédentes **caractérisé en ce que** le précurseur catalytique contient la totalité des éléments des groupes GVIB et s'ils sont présents, la totalité des éléments du groupe VIII.

**4.** Procédé selon l'une des revendications précédentes **caractérisé en ce que** le succinate de dialkyle est le succinate de diméthyle.

**5.** Procédé selon l'une des revendications précédentes **caractérisé en ce que** l'acide carboxylique est l'acide citrique.

**6.** Procédé selon l'une des revendications précédentes **caractérisé en ce que** les étapes 1) et/ou 2) sont réalisées en présence d'eau et/ou d'éthanol.

**7.** Procédé selon l'une des revendications précédentes **caractérisé en ce que** les étapes de maturation sont réalisées à une température entre 17 et 60°C.

**8.** Procédé selon l'une des revendications précédentes **caractérisé en ce que** le séchage de l'étape 1 est réalisé à une température comprise entre 100 et 180°C.

**9.** Procédé selon l'une des revendications précédentes **caractérisé en ce que** le séchage de l'étape 2 est réalisé est réalisé à une température comprise entre 50 et 160°C.

**10.** Procédé selon l'une des revendications précédentes **caractérisé en ce que** la solution de l'étape 1 et/ou de l'étape 2 contient moins un composé de phosphore, et éventuellement l'acide acétique.

**11.** Procédé selon l'une des revendications précédentes **caractérisé en ce que** l'étape 2) est réalisée en présence d'acide acétique.

**12.** Procédé selon l'une des revendications précédentes **caractérisé en ce qu'**il comporte une étape finale de sulfuration.

**13.** Procédé d'hydrotraitement et/ou d'hydroconversion de charges hydrocarbonées en présence d'un catalyseur préparé par le procédé selon la revendication 12.

**14.** Procédé selon la revendication 13 dans lequel l'hydrotraitement est une hydrodésulfuration, une hydrodéazotation, une hydrodémétallation, ou une hydrogénation des aromatiques.


**Patentansprüche**

**1.** Verfahren zur Herstellung eines Hydrotreating- und/oder Hydrokonversionskatalysators aus einer Katalysatorvorstufe, die einen Träger auf Basis von Aluminiumoxid und/oder Siliciumdioxid-Aluminiumoxid und/oder Zeolith und mindestens ein Element der Gruppe VIB und gegebenenfalls mindestens ein Element der Gruppe VIII umfasst, wobei das Verfahren das Imprägnieren der Vorstufe mit einer Lösung eines Bernsteinsäure-di-C1-C4-alkylesters umfasst, **dadurch gekennzeichnet, dass** es die folgenden Schritte umfasst:

1) Imprägnieren (Schritt 1) der getrockneten, calcinierten oder regenerierten Vorstufe mit mindestens einer Lösung, die mindestens eine Carbonsäure, die von Essigsäure verschieden ist, enthält, dann Reifen und Trocknen bei einer Temperatur von weniger als 200 °C, gegebenenfalls mit anschließender Wärmebehandlung bei einer Temperatur von weniger als 350 °C,
2) anschließendes Imprägnieren (Schritt 2) mit einer Lösung, die mindestens einen Bernsteinsäure-di-C1-C4-alkylester umfasst, dann Reifen und Trocknen bei einer Temperatur von weniger als 200 °C ohne nachfolgenden Calcinierungsschritt,

und der Katalysatorvorläufer und/oder die Lösung von Schritt 1 und/oder die Lösung von Schritt 2 Phosphor enthält.

**2.** Verfahren nach Anspruch 1, **dadurch gekennzeichnet, dass** es sich bei der Katalysatorvorstufe um einen regenerierten Katalysator handelt.

**3.** Verfahren nach einem der vorhergehenden Ansprüche, **dadurch gekennzeichnet, dass** die Katalysatorvorstufe die gesamten Elemente der Gruppen GVIB und, sofern sie vorliegen, die gesamten Elemente der Gruppe VIII enthält.

**4.** Verfahren nach einem der vorhergehenden Ansprüche, **dadurch gekennzeichnet, dass** es sich bei dem Bernsteinsäuredialkylester um Bernsteinsäuredimethylester handelt.

**5.** Verfahren nach einem der vorhergehenden Ansprüche, **dadurch gekennzeichnet, dass** es sich bei der Carbonsäure um Citronensäure handelt.

**6.** Verfahren nach einem der vorhergehenden Ansprüche, **dadurch gekennzeichnet, dass** die Schritte 1) und/oder 2) in Gegenwart von Wasser und/oder Ethanol durchgeführt werden.

**7.** Verfahren nach einem der vorhergehenden Ansprüche, **dadurch gekennzeichnet, dass** die Reifungsschritte bei einer Temperatur zwischen 17 und 60 °C durchgeführt werden.

**8.** Verfahren nach einem der vorhergehenden Ansprüche, **dadurch gekennzeichnet, dass** die Trocknung von Schritt 1 bei einer Temperatur zwischen 100 und 180 °C durchgeführt wird.

**9.** Verfahren nach einem der vorhergehenden Ansprüche, **dadurch gekennzeichnet, dass** die Trocknung von Schritt 2 bei einer Temperatur zwischen 50 und 160 °C durchgeführt wird.

10. Verfahren nach einem der vorhergehenden Ansprüche, **dadurch gekennzeichnet, dass** die Lösung von Schritt 1 und/oder von Schritt 2 mindestens eine Phosphorverbindung und gegebenenfalls Essigsäure enthält.

11. Verfahren nach einem der vorhergehenden Ansprüche, **dadurch gekennzeichnet, dass** Schritt 2) in Gegenwart von Essigsäure durchgeführt wird.

12. Verfahren nach einem der vorhergehenden Ansprüche, **dadurch gekennzeichnet, dass** es einen abschließenden Sulfurierungsschritt umfasst.

13. Verfahren zum Hydrotreating und/oder zur Hydrokonversion von Kohlenwasserstoff-Einsatzstoffen in Gegenwart eines durch das Verfahren nach Anspruch 12 hergestellten Katalysators.

14. Verfahren nach Anspruch 13, bei dem es sich bei dem Hydrotreating um eine Hydrodesulfurierung, eine Hydrodenitrogenierung, eine Hydrodemetallisierung oder eine Aromatenhydrierung handelt.

**Claims**

1. Process for the preparation of a hydrotreating and/or hydroconversion catalyst from a catalytic precursor comprising a support based on alumina and/or on silica/alumina and/or on zeolite, and comprising at least one element from group VIB and optionally at least one element from group VIII, the said process comprising the impregnation of the said precursor with a solution of a di($C_1$-$C_4$ alkyl) succinate, **characterized in that** it comprises the following stages:

   1) impregnating (stage 1) the said dried, calcined or regenerated precursor with at least one solution containing at least one carboxylic acid other than acetic acid, then maturing and drying at a temperature of less than 200°C, optionally followed by a heat treatment at a temperature of less than 350°C,
   2) followed by impregnating (stage 2) with a solution containing at least one di($C_1$-$C_4$ alkyl) succinate, then maturing and drying at a temperature of less than 200°C, without a subsequent calcination stage,

   and the catalytic precursor and/or the solution of stage 1 and/or the solution of stage 2 contains phosphorus.

2. Process according to Claim 1, **characterized in that** the catalytic precursor is a catalyst which has been regenerated.

3. Process according to either of the preceding claims, **characterized in that** the catalytic precursor contains all of the elements of groups GVIB and, if they are present, all of the elements of group VIII.

4. Process according to one of the preceding claims, **characterized in that** the dialkyl succinate is dimethyl succinate.

5. Process according to one of the preceding claims, **characterized in that** the carboxylic acid is citric acid.

6. Process according to one of the preceding claims, **characterized in that** stages 1) and/or 2) are carried out in the presence of water and/or of ethanol.

7. Process according to one of the preceding claims, **characterized in that** the maturing stages are carried out at a temperature of between 17°C and 60°C.

8. Process according to one of the preceding claims, **characterized in that** the drying in stage 1) is carried out at a temperature of between 100°C and 180°C.

9. Process according to one of the preceding claims, **characterized in that** the drying of stage 2) is carried out at a temperature of between 50°C and 160°C.

10. Process according to one of the preceding claims, **characterized in that** the solution of stage 1) and/or of stage 2) contains at least one phosphorus compound, and optionally acetic acid.

11. Process according to one of the preceding claims, **characterized in that** stage 2) is carried out in the presence of acetic acid.

12. Process according to one of the preceding claims, **characterized in that** it comprises a final sulfidation stage.

13. Process for the hydrotreating and/or hydroconversion of hydrocarbon feedstocks in the presence of a catalyst prepared by the process according to Claim 12.

14. Process according to Claim 13, in which the hydrotreating is a hydrodesulfurization, a hydrodenitrogenation, a hydrodemetallization, or hydrogenation of aromatics.

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0870003 A
- WO 2006077326 A
- WO 11080407 A
- WO 05035691 A
- EP 0482817 A
- WO 201180407 A

**Littérature non-brevet citée dans la description**

- **GRIBOVAL ; BLANCHARD ; PAYEN ; FOURNIER ; DUBOIS.** *Catalysis Today,* 1998, vol. 45, 277
- **M. T. POPE.** Heteropoly and Isopoly oxometalates. Springer Verlag, 8
- **GRIBOVAL ; BLANCHARD ; GENGEMBRE ; PAYEN ; FOURNIER ; DUBOIS ; BERNARD.** *Journal of Catalysis,* 1999, vol. 188, 102
- **CH. BAERLOCHER ; L. B. MC CUSKER ; D.H. OISON.** Atlas of Zeolite Structure Types. Elsevier, 2007, Elsevier, 2007
- **C. REICHARDT.** Solvents and Solvent Effects in Organic Chemistry. Wiley-VCH, 2003, 472-474